# EUROPÄISCHE PATENTSCHRIFT

(11) **EP 2 345 450 B1**
(45) Veröffentlichungstag und Bekanntmachung des Hinweises auf die Patenterteilung: **07.09.2016**
(21) Anmeldenummer: 10150868.7
(22) Anmeldetag: 15.01.2010
(51) Int. Cl.: A61M 25/00, A61M 5/31

(54) **Applikations-Set umfassend einen Applikator und eine Schlauchhülle für den Applikator, und Implantationsbesteck**
Application set comprising an Applicator and a tubular casing for the applicator, and implantation instruments
Kit d'application comprenant un Applicateur et une gaine en boyau pour l'applicateur, et trousse d'application

(43) Veröffentlichungstag der Anmeldung: 20.07.2011
(73) Patentinhaber: Co.Don AG, 14513 Teltow (DE)
(72) Erfinder: Oppat, Julia, 12559 Berlin (DE); Methner, Vilma, 14532 Kleinmachnow (DE); Giesemann, Petra, 12279 Berlin (DE); Libera-Körner, Jeanette, 12487 Berlin (DE)
(74) Vertreter: Simandi, Claus

(56) Entgegenhaltungen:
- EP-A1- 2 106 821
- WO-A1-02/094334
- WO-A1-2004/056414
- DE-A1- 2 624 328
- DE-A1- 4 137 132
- GB-A- 2 274 991
- US-A- 3 612 038
- US-A- 4 200 096
- US-A- 4 509 945
- US-A1- 2003 236 500

## Beschreibung

Die vorliegende Erfindung betrifft einen Applikator zur zumindest temporären Aufnahme und Applikation von Substanzen, insbesondere zur Applikation einer Zellhaltigen und/oder gelförmigen oder flüssigen Substanz in tierische oder menschliche Organe oder Gewebe.

Außerdem betrifft die Erfindung eine Schlauchhülle zur Befestigung am erfindungsgemäßen Applikator und Schutz desselben.

Die Erfindung betrifft in ihrer Gesamtheit ein Applikations-Set, welches den erfindungsgemäßen Applikator und die erfindungsgemäße Schlauchhülle umfasst, sowie durch ein Implantationsbesteck, welches wenigstens einen erfindungsgemäßen Applikator und/oder ein erfindungsgemäßes Applikations-Set aufweist.

Zur Einbringung von medizinischen Substanzen in biologische Organe oder Gewebe, sind Flexülen, Braunülen, Kanülen oder einfache Schläuche bekannt. Die sogenannten Flexülen und Braunülen weisen eine Kanüle auf, über die ein Schlauch schiebbar ist, so dass der Schlauch mitsamt der Kanüle in das Organ oder Gewebe eingeführt, die Kanüle aus dem Schlauch herausgezogen und eine Substanz durch den Schlauch in das Gewebe eingebracht werden kann. Aufgrund der temporären Anordnung des Schlauches auf der Kanüle kann der Schlauch nicht als Container zur Aufnahme und Einbringung einer Substanz in das Gewebe bzw. in ein Gefäßlumen genutzt werden. Außerdem besteht bei den Flexülen oder Braunülen die Gefahr, dass bei einem Eindringen in tiefere Lumen oder tiefer liegendes Gewebe des menschlichen oder tierischen Körpers der Schlauch nicht an der richtigen Stelle positioniert ist und/oder dass die Position des Schlauchendes nicht eindeutig bestimmbar oder kontrollierbar ist, da derartige Systeme zu kurz und die Materialien der Schläuche und Kanülen für die Video-assistierte Arthroskopie und Endoskopie nicht geeignet sind und/oder weil die Materialien nicht formbar sind.

Im Stand der Technik werden Applikatoren oder Katheter wie folgt beschrieben:
WO 2004/056414A1 beschreibt verformbare Katheter zur urinären Katheterisierung. Die besagten Katheter können einen Metalldraht enthalten, um die per Hand geformte Ausrichtung des Katheters zu fixieren.
DE 41 37 132 A1 beschreibt Vorrichtungen zur direkten Absaugung und Spülung im Nasen-, Nasennebenhöhlen-, Mund-, Rachen- und Luftröhrenbereich direkt im Bereich der Wunde. Hierbei bestehen die Vorrichtungen aus einem rohrförmigen Körper zur Absaugung und Spülung, wobei der rohrformige Körper aus inertem Kunststoff besteht, in dem eine metallische Verstärkung in Form von Metalldrähten oder -bändern eingebettet ist.
WO 2002/094334 A1 beschreibt ein verlängertes medizinisches Gerät zum Einfügen in ein Blutgefäß, Organ oder in eine Körperhöhle, zwecks Platzierung eines therapeutischen oder diagnostischen, distalen Segments, welches verformbar ist, um es den anatomischen Gegebenheiten bei Raum-Temperatur anzupassen.
US 3 612 038 A beschreibt vorgeformt sterilisierbare Katheter in Anordnung mit einer Sterilfolie. Hierbei ist ein verform- und entfernbarer Draht im Lumen des Katheters aufgenommen, welcher durch Erhitzung und Erkaltung des Drahtes die Form des Katheters vörfixiert und daraufhin entfernt werden kann.
GB 2 274 991 A beschreibt einen Embryo-Austausch Katheter mit einer Röhre, welche durch zwei separate Lumen gekennzeichnet ist. Hierbei dient ein Lumen der Aufnahme des Embryos und das zweite Lumen der Verformung und Kontrolle des Katheters.
US 2003/236500 A1 beschreibt eine Vorrichtung für Injektionszwecke von Lösungen, welche aus pulverigen Medikamenten in sterilem Wasser hergestellt wird, gekennzeichnet durch eine Flügelkanüle.
US 4 200 096 A beschreibt eine Vorrichtung zum Injizieren von Flüssigkeiten in Blutgefäße, wobei die besagte Flüssigkeit kein Blut ist. Hierbei wird eine biegsame Nadel verwendet, welche mit einer Versorgungseinheit fest verbunden ist und durch eine luft- und blutundurchlässige Membran, welche aber Flüssigkeiten passieren können, versehen ist.
DE 26 24 328 A1 beschreibt eine medizinische Verabreichungsnadelanordnung mit Filtereinrichtung.
US 4 509 945 A beschreibt Katheter zum Katheterisieren von Venen mit einem J-förmigen Führungsdraht, welcher entfernbar ist.

Zur Injektion von Substanzen, wie dreidimensionale Zelltransplantate in Form von kleinen Kügelchen, sogenannten Spheroiden, in Gewebe werden oftmals einfache Spritzen benutzt, wobei jedoch nicht gewährleistet ist, dass alle in der Spritze und daran angeschlossener Kanüle aufgenommene Spheroide auch tatsächlich bis zum Behandlungsort transportiert werden, da nach vollständiger Entleerung des Spritzenvolumens Spheroide noch in der Kanüle verbleiben können. Das heißt, es besteht die Gefahr des Verlustes des Medikamentes zwischen der Befüllung des Schlauches und der Injektion. Dies ist bedingt durch die Geometrie der Systeme, die Ecken und Kanten aufweist, in welchen die Substanzen und Partikel hängen bleiben können.

Zur Einbringung von medizinischen Substanzen in den menschlichen oder tierischen Körper werden die Substanzen in Transportgefäßen wie Spritzen oder Röhrchen transportiert. Der Arzt muss zur Anwendung der Substanzen ggf. selbige zunächst aus dem Röhrchen in eine Spritze überführen. Dazu benötigt er eine Kanüle. Mittels der Kanüle kann er die Substanz auch in den Körper einbringen. Befindet sich die Substanz bereits in einer Spritze, wie bei z.B. Impfstoffen vorgesehen, dann muss er nur eine Kanüle auf die Spritze aufsetzen und kann die Einbringung in den menschlichen oder tierischen Körper vornehmen. Beide Verfahren bergen das Risiko einer bakteriellen Infektion der medizinischen Substanz durch Verunreinigungen der Kanülen oder unreine Handhabung der Kanülen. Bei der Überführung aus einem Röhrchen in eine Spritze tritt weiterhin ein Verlust an medizinischer Substanz auf, da die Substanz aus dem Röhrchen heraus nicht vollständig aufgenommen und abgegeben werden kann.

Bei der Behandlung eines Organ- oder Gewebedefektes durch einen nicht-geradlinigen Behandlungskanal würde sich der Einsatz von Filterhalmen anbieten, die zur Entnahme und Filtration von Medikamenten aus Ampullen mit einem Schlauch und einer Aufnahmeeinrichtung zur Aufnahme einer Spritzendüse ausgestattet sind, da derartige Filterhalme flexibel verformbar sind. Nachteilig an diesen Filterhalmen ist jedoch, dass aufgrund ihrer flexiblen Verformbarkeit nicht immer eine exakte Positionierung am Behandlungsort realisiert werden kann, da sich die Flexüle aufgrund ihrer Elastizität gegebenenfalls abweichend vom Behandlungskanal im Gewebe erstrecken würde. Außerdem besteht die Gefahr des Verlustes des Medikamentes zwischen der Befüllung des Schlauches und der Injektion. Weiterhin besteht die Gefahr der Verschmutzung bzw. Beschädigung des Schlauches vor oder bei Einführung in das Gewebe. Ein Filterhalm ist dabei nicht zum Transport der Substanzen und Partikeln ausgelegt, so die Substanzen über eine Spritze durch den Filterhalm appliziert werden müssen. Die Spritze, in der die Substanzen lagern, weist wie beschrieben eine Geometrie mit Ecken und Kanten auf, die keine vollständige Abgabe von Substanzen und Partikeln erlaubt.

Der Erfindung liegt somit die Aufgabe zugrunde, einen Applikator sowie eine Ein-richtung zum Schutz des Applikators zur Verfügung zu stellen, welche kostengünstig und in einfacher Weise eine sichere, sterile, zuverlässige, vollständige und kontrollierbare Einbringung von Substanzen in Organe und Gewebe ermöglichen und gleichzeitig den Transport erleichtem.

Diese Aufgabe wird durch das erfindungsgemäße Applikations-Set, umfassend den erfindungsgemäßen Applikator und die erfindungsgemäße Schlauchhülle nach Anspruch 1 gelöst.

Vorteilhafte Ausgestaltungen des erfindungsgemäßen Applikations-Sets und damit des erfindungsgemäßen Applikators und der erfindungsgemäßen Schlauchhülle, sind in den Unteransprüchen 2 bis 12 angegeben.

Erfindungsgemäß wird ein Applikator zur zumindest temporären Aufnahme und Applikation von Substanzen, insbesondere zur Applikation einer Zell-haltigen und/oder gelförmigen oder flüssigen Substanz in tierische oder menschliche Organe oder Gewebe zur Verfügung gestellt, wobei der Applikator einen manuell biegbaren Schlauch umfasst und mit dem Schlauch mechanisch wenigstens ein manuell plastisch biegbares Biegeelement verbunden ist, mit welchem nach Einwirkung eines auf den Applikator wirkenden Biegemomentes der Schlauch in einem verformten Zustand haltbar ist.

Der Applikator kann auch zur zumindest temporären Aufnahme und Separation, Filterung, Aufreinigung, Lagerung, Transport und medizinischen oder technischen Applikation von Substanzen, Gemischen, die gegebenenfalls auch als Pulver ausgeführt sind, oder Partikeln, insbesondere zur Applikation einer Zelltransplantat-haltigen Lösung, in vivo oder ex vivo, in Organe oder Gewebe des menschlichen oder tierischen Körpers genutzt werden.

Der erfindungsgemäße Applikator ist insbesondere vorgesehen zur Aufnahme von Zelltransplantaten und zum arthroskopischen Einbringen dieser in Organe oder Gewebe des menschlichen oder tierischen Körpers, wie z. B. Knorpelgewebe in Kniegelenken, Hüftgelenken oder Bandscheiben. Der Schlauch des Applikators kann aus einem Material bestehen oder eine Beschichtung aufweisen, die die Anhaftung von Substanzen oder Partikeln fördert oder verhindert. Der erfindungsgemäße Applikation ist vorzugsweise zumindest auf seiner Innenseite aus einem Material gefertigt, welches eine auf Grund chemischer und/ oder biologischer Reaktion hervorgerufene Anhaftung der Substanz bzw. der Zelltransplantate am Schlauch verhindert. Der Schlauch kann z. B. aus einem durchsichtigen Material wie zum Beispiel Teflon, PTFE, Silikon oder Polyurethan gefertigt sein und einen Innendurchmesser von 0,1 mm bis 2,5 mm, vorzugsweise 0,8 mm bis 1,5 mm und insbesondere 1,3 mm aufweisen. Er kann je nach zu behandelndem Defekt eine Länge von 7,5 mm bis 500 mm, vorzugsweise 150 mm bis 250 mm haben.

In einer bevorzugten Ausführungsform weist der Applikator eine Aufnahmeeinrichtung zum Anschluss einer Spritzendüse auf, so dass bei Druckerhöhung bzw. Druckverringerung z.B. in einer an die Anschlusseinrichtung angeschlossenen Spritze über die Anschlusseinrichtung der Schlauch mit der Substanz teilweise oder vollständig entleer- bzw. befüllbar ist. Die Erzeugung eines Unterdrucks in der Spritze bewirkt ein Einsaugen der Substanz in den Schlauch und in das Spritzenvolumen. Die Erzeugung eines Überdrucks In der Spritze bewirkt ein Ausstoßen der Substanz aus dem Spritzenvolumen und aus dem Schlauch.

Vorzugsweise ist die mechanische Verbindung des Schlauches mit dem Biegeelement dadurch realisiert, dass das Biegeelement im Schlauch aufgenommen ist. Dabei kann das Biegeelement in der Schlauchwand angeordnet sein. Das Biegeelement ist in dieser Ausführungsform somit nicht im Hohlraum des Schlauches angeordnet, sondem vollständig vom Material der Schlauchwand umgeben.

Es ist dabei insbesondere vorgesehen, dass das Biegeelement in einem komplementär zu seiner Form gestalteten Hohlraum im Schlauch angeordnet ist, der bis zum proximalen Ende des Schlauches verläuft, wobei das Biegeelement vor dem proximalen Ende des Schlauches endet und der verbleibende komplementäre Hohlraum verschlossen ist. Der komplementäre Hohlraum, in dem das Biegeelement angeordnet ist, ist dabei nicht der von der Schlauchwand gebildete Innenraum des Schlauches. Der verbleibende, nicht vom Biegeelement ausgefüllte komplementäre Hohlraum ist vorzugsweise mit einer Klebemasse ausgefüllt. Das Ende des Biegeelementes ist somit zum proximalen Ende des Schlauches beabstandet. Der Vorteil dieser Ausgestaltung liegt in der einfachen Fertigung des Schlauches mit Biegeelement, bei der das Biegeelement in den durchgängigen komplementären Hohlraum eingeschoben wird und der nicht vom Biegeelement ausgefüllte komplementäre Hohlraum mit Klebemasse verschlossen wird.

Das Biegeelement ist wenigstens ein Draht oder ein anderes biegsames Material. Vorzugsweise ist das Biegeelement aus Titan oder einer geeigneten Titanlegierung. Insbesondere die Ausgestaltung des Biegeelementes als ein Vollmaterial-Titan-Draht weist den Vorteil einer einfachen Fertigung sowie der Sichtbarkeit des Drahtes in einer MRT-Aufnahme auf. Das heißt, dass das Biegeelement eine MRT-Aufnahme nicht stört, jedoch visuell in der MRT-Aufnahme erkennbar ist, so dass die Position des Applikators bei einer Operation und gleichzeitiger MRT-Aufnahme erkennbar und gegebenenfalls korrigierbar ist.

Durch das Biegeelement ist der Schlauch des Applikators derart vorverformbar, dass er im Wesentlichen der Form eines gegebenenfalls nicht-linearen Behandlungskanals anpassbar ist. Das heißt, dass der Schlauch des Applikators manuell in eine passende Form bringbar ist, so dass er mit geringstmöglicher Beschädigung oder Verschiebung des Organs oder Gewebes in dieses einbringbar ist und mit größter Genauigkeit am Ort der Behandlung positionierbar ist.

In weiterer vorteilhafter Ausführungsform umfasst der Applikator wenigstens einen mit dem Schlauch strömungstechnisch verbundenen Filter, der gasdurchlässig ist. Dabei ist der Filter vorzugsweise flüssigkeitsundurchlässig und/oder undurchlässig für Zelltransplantate mit einem Durchmesser von 0,2 µm bis zu 650 µm. Er ist dabei bevorzugt aus einem biokompatiblen Material gefertigt, z.B. aus PTFE, Polyester oder PVDF, und weist eine Porengröße auf, die verhindert, dass die Zelltransplantate durch ihn hindurch gelangen oder auch festkleben können. Eine bevorzugte Porengröße beträgt zwischen 0,2 µm bis 200 µm. Der Filter ermöglicht den Verbleib der Substanz oder Partikel im Schlauch zu Transportzwecken und verhindert die Zerstörung der Zelltransplantate beim Einsaugen in den Schlauch und außerdem ein Klebenbleiben oder Verstopfen des Filters, da sich die Spheroide am Filter im Wesentlichen in Längsrichtung des Schlauches aufreihen.

Der Filter ist dabei bevorzugt am distalen Ende des Schlauches angeordnet, um eine vollständige Befüllung des Schlauches mit der Substanz bzw. mit den Zelltransplantaten zu ermöglichen. In einer alternativen Ausführungsform kann der Filter auch in der Aufnahmeeinrichtung angeordnet sein, so dass aufgrund von Unterdruck in den Schlauch eingesogene Substanz auch bis in die Aufnahmeeinrichtung gelangt. Der Filter kann auch mittig oder an anderen Zwischenpositionen im Schlauch angeordnet sein, um den Schlauch nur hälftig oder entsprechend abweichend zu befüllen. Der Filter verhindert die Strömung der Substanz bzw. der Zelltransplantate in eine an den Applikator angesetzte Saugspritze, wobei eine Trägerflüssigkeit durch den Filter in die Saugspritze gesogen wird.

Der Schlauch des Applikators kann zumindest teilweise mit einer zu injizierenden Substanz bzw. mit den zu injizierenden Zelltransplantaten gefüllt sein. Das heißt, dass der erfindungsgemäße Applikator nicht nur dazu genutzt werden kann, am Ort der Operation mit der Substanz bzw. mit den Zelltransplantaten befüllt zu werden und anschließend diese Substanz in Organe oder Gewebe abzugeben, sondern dass er auch an einem vom Empfänger entfernten Ort mit der Substanz bzw. den Zelltransplantaten befüllt, zum Operationsort transportiert und dort in das Organ oder Gewebe die Substanz bzw. die Zelltransplantate abgeben kann.

Erfindungsgemäß wird außerdem eine Schlauchhülle zur Verfügung gestellt, die einen Mantelbereich sowie eine mit dem Mantelbereich mechanisch verbundene Befestigungseinrichtung zur mechanischen Befestigung der Schlauchhülle an dem erfindungsgemäßen Applikator aufweist. Die Befestigungseinrichtung kann am Schlauch und/oder an der Aufnahmeeinrichtung des Applikators befestigt werden. Der Mantelbereich bildet einen Hohlraum aus, der zur Aufnahme des Applikators, insbesondere zur Aufnahme des Schlauches, geeignet ist, um den Schlauch vor Beschädigung und Verschmutzung zu schützen. Vorzugsweise sind dabei der Durchmesser, die Länge sowie die Elastizität der Schlauchhülle und des Schlauches derart bemessen, dass zwischen Schlauchhülle und Schlauch eine leichte Presspassung besteht. Dadurch wird zum einen eine ungewollte Lösung der Schlauchhülle vom Schlauch verhindert, jedoch gewährleistet, dass die Schlauchhülle mit manuell aufbringbarer Zugkraft vom Schlauch abgezogen werden kann und zum anderen, dass ein fester, wasserdichter, proximaler Verschluss des Applikators durch die Schlauchhülle erreicht wird. Die Schlauchhülle kann jedoch auch im Vergleich zum Applikator einen größeren Durchmesser des Hohlraumes aufweisen oder auch länger als der Applikator sein.

Der Mantelbereich der Schlauchhülle ist zumindest abschnittsweise aus einem durchsichtigen Material hergestellt, wie zum Beispiel aus Polyvinylchlorid, Polyimind, Teflon oder PTFE. Vorzugsweise ist der Mantel vollständig transparent.

Der die Form eines Hohlzylinders aufweisende Mantelbereich der Schlauchhülle ist am proximalen Ende mit einem Stopfen verschlossen. Das proximale Ende der Schlauchhülle liegt dem Schlauchhüllenende gegenüber, an welchem die Befestigungseinrichtung angeordnet ist. Der Vorteil der Schlauchhülle liegt in ihrer einfachen Herstellung, nämlich in der Anordnung einer Befestigungseinrichtung an einem schlauchförmigen Mantelbereich und Verschluss des schlauchförmigen Mantelbereichs durch einen Stopfen. Die Schlauchhülle ist über den gesamten Schlauch des Applikators schiebbar, wobei sich die Luft in der Schlauchhülle seitlich am Schlauch aus der Schlauchhülle herausdrückt und/oder durch die Substanz im Schlauch und durch den gasdurchlässigen Filter herausdrückt. Dadurch wird ein Verschluss des Schlauches und gleichzeitiger Schutz des Schlauches gegen Verschmutzung und Beschädigung zur Verfügung gestellt.

Bei Anwendung des Stopfens ist dieser bevorzugt aus Silikon zu fertigen.

Bei der Auswahl eines elastischen Materials des Stopfens, wie z. B. Silikon, gewährleistet die Elastizität des Stopfens eine ständige Anlage des angedrückten Applikatorendes an der Schlauchhülle, so dass ein Austreten der Substanz oder Partikel proximal verhindert wird. Es ist auch möglich; den Stopfen aus einem nicht elastischen Material und/oder einem Flüssigkeits-aufsaugendem Material herzustellen oder aus einem gasdurchlässigen Material herzustellen.

Die Befestigungseinrichtung der Schlauchhülle ist vorzugsweise ein Bestandteil eines Luer-Locks. Dieses Luer-Lock dient zur mechanischen Verbindung, dem Verschließen und Fixierung der Schlauchhülle mit dem Schlauch bzw. mit der Aufnahmeeinrichtung des Applikators, wobei der Schlauch oder die Aufnahmeeinrichtung das jeweils andere Bestandteil des Luer-Locks aufweist. Alternativ kann statt eines Luer-Locks auch eine mechanische Verbindung mittels eines Bajonett-Verschlusses oder eines Gewindes oder einer mechanische Einpressung oder Verklemmung realisiert sein. Sämtliche Verschlüsse können z. B. aus Polypropylen, Polyehtylen oder Polytetralfluorethylen bestehen. Vorzugsweise sind die Verschlüsse aus Polysterol herzustellen. Weiterhin kann die Befestigung der Schlauchhülle mit dem Schlauch gasdicht oder nicht gasdicht realisiert sein. Vorzugsweise ist diese Verbindung gasdicht, um eine kontamination des Schlauches und ein Austreten von Substanz zu verhindern.

Durch die Erfindung wird ein Appükations-Set zur Verfügung gestellt, welches einen erfindungsgemäßen Applikator und eine erfindungsgemäße Schlauchhülle und einen distalen Verschluss des Schlauches umfasst, wobei der Schlauch des Applikators zumindest teilweise vom Mantelbereich der Schlauchhülle umschlossen ist. Vorzugsweise ist der Schlauch des Applikators vollständig in der Schlauchhülle aufgenommen und die Schlauchhülle ist mit der Befestigungseinrichtung am Applikator befestigt, so dass die Schlauchhülle den Schlauch des Applikators vor Verschmutzung und Beschädigung schützt und diesen vollständig verschließt. Ein derartiges Applikations-Set entspricht einem arthroskopischen Injektionssystem, vorzugsweise zur Injektion von Zelltransplantaten mit einem Durchmesser von 400 bis zu 1200 µm.

Außerdem wird erfindungsgemäß ein Implantations- oder Transplantationsbesteck zur Verfügung gestellt, weiches mindestens einen erfindungsgemäßen Applikator oder mindestens ein erfindungsgemäßes Applikations-Set umfasst, wobei das Implantationsbesteck eine sterile und im Wesentlichen hermetisch abdichtende Verpackung aufweist und der Applikator oder das Applikations-Set in der Verpackung aufgenommen ist. Das heißt, dass in der Verpackung der Applikator mit oder ohne Schlauchhülle aufgenommen sein kann. Auch können weitere zusätzliche Applikationshilfen bzw. Aufnahmehilfen im Set enthalten sein, die in Verbindung mit dem Applikator für dessen Bedienung verwendet werden können.

In dem Beispiel, in dem sich in der Verpackung der Applikator mit Schlauchhülle befindet und somit ein Applikations-Set in der Verpackung aufgenommen ist, ist zur Benutzung des Applikators die Verpackung zu entfernen und der Applikator zu entnehmen. Danach ist eine Saugspritze an den Applikator anzusetzen und das proximale Ende des Schlauches in die substanz- oder partikelhaltige Lösung oder Gemisch zu halten. Dann ist durch Erzeugung eines Unterdruckes in der Saugspritze der Applikator mit Substanz bzw. Zelltransplantaten zu befüllen und anschließend zum Behandlungsort bzw. zum Operationsort zu verbringen. Dies kann in geeigneten Transportsystemen für Implantate oder lebende Transplantate erfolgen. Wenn notwendig, ist am Operationsort durch Einleitung eines Biegemomentes der Schlauch des Applikators in eine zur Einführung in das menschliche oder tierische Organ oder Gewebe passende Form zu bringen. Der Schlauch des Applikators ist in das Organ oder Gewebe einzubringen und durch Erzeugung eines Überdruckes mittels einer an dem Applikator angeordneten Spritze oder eines anderen Druck erzeugenden Systems ist die Substanz bzw. das Zelltransplantat aus dem Applikator in das Organ oder Gewebe, welches zum Beispiel Knorpelgewebe sein kann, zu injizieren. Der Vorteil liegt insbesondere darin, dass durch die Anwendung der Schlauchhülle ein Schutz des Applikators vor Verschmutzung und Beschädigung gewährleistet ist und durch das Biegeelement der Schlauch plastisch in gebogener Form haltbar ist, wobei das plastisch verbogene Biegeelement einer elastizitätsbedingten Rückstellkraft des Schlauches zumindest teilweise entgegenwirkt. Das heißt, dass nur eine geringe Rückfederung des Applikatorschlauches nach Einwirkung des Biegemomentes auf diesen auftritt.

Das erfindungsgemäße Applikations-Set kann alternativ auch beispielhaft derart genutzt werden, dass der Applikator mit einer Substanz durch Ansetzen einer Saugspritze und Erzeugung eines Unterdruckes befüllt wird, danach der Schlauch des Applikators durch Herüberziehen der Schlauchhülle und Schließen der Befestigungseinrichtung zwischen Schlauchhülle und Applikator geschlossen wird und danach das Applikations-Set gelagert und/oder zum Behandlungsort transportiert wird. Dort erfolgt eine Entfernung der Schlauchhülle vom Applikator und gegebenenfalls eine Verformung des Schlauches zur Anpassung an die Form und Lage des zu behandelnden Organs oder Gewebes. Nach Einbringung des Applikatorschlauches kann die im Applikatorschlauch aufgenommene Substanz bzw. können die im Schlauch aufgenommenen Zelltransplantate in das menschliche oder tierische Organ oder Gewebe injiziert werden.

Vorzugsweise dient der erfindungsgemäße Applikator zur Behandlung von Knorpeldefekten, wobei der erfindungsgemäße Applikator zur Injektion einer Substanz bzw. von Zelltransplantaten an oder in einen zu behandelnden Knorpel genutzt wird.

Die vorliegende Erfindung wird im Folgenden anhand der beiliegenden Zeichnungen beschrieben. Es zeigt:
- Figur 1: einen erfindungsgemäßen Applikator in Schnittdarstellung von der Seite,
- Figur 2: einen Schnitt durch den Schlauch des Applikators gemäß des in Figur 1 angedeuteten Schnittverlaufs A-A,
- Figur 3: eine erfindungsgemäße Schlauchhülle in Schnittdarstellung von der Seite,
- Figur 4: ein aus Applikator und Schlauchhülle zusammengesetztes Applikations-Set in Schnittdarstellung von der Seite, wobei der Schlauch des Applikators und die Schlauchhülle gebrochen dargestellt sind.

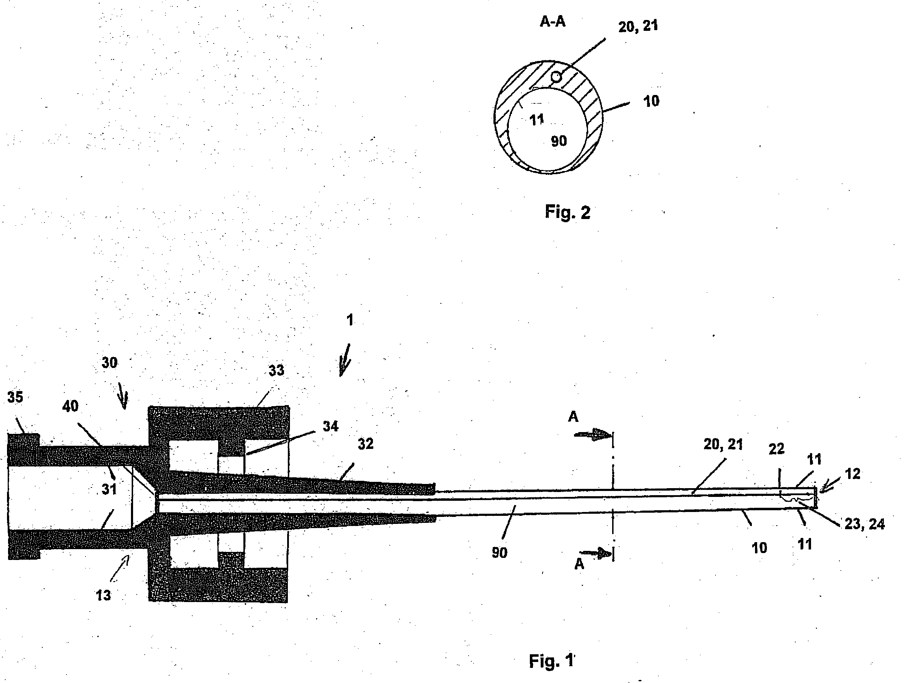

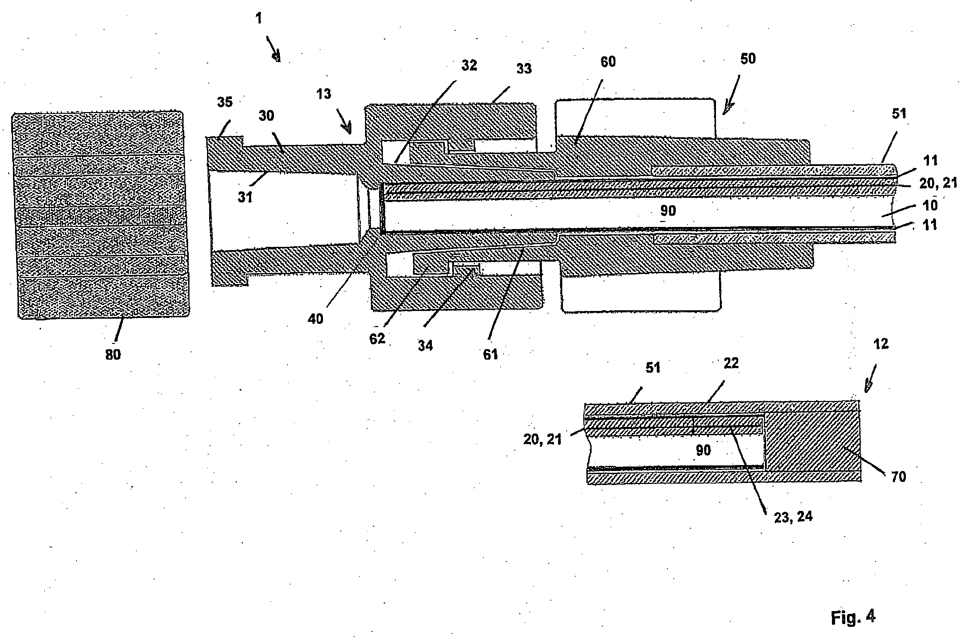

Der in Figur 1 dargestellt Applikator 1 umfasst als wesentliche Baueinheiten einen Schlauch 10, ein im Schlauch 10 angeordnetes Biegeelement 20 sowie eine am Schlauch distal angeordnete Aufnahmeeinrichtung 30. Der Schlauch 10 ist mit einem Außenkonus 32 fest verbunden. Am distalen Ende des Schlauches 10 ist ein Filter 40 angeordnet. Der Filter 40 verschließt somit das distale Ende 13 des Schlauches 10. Die Aufnahmeeinrichtung 30 weist einen Innenkonus 31 auf, der zur Aufnahme einer Spritzendüse geeignet ist. An dem den Innenkonus 31 ausgestaltenden Formelement ist radial abstehend ein äußeres Verriegelungselement 35 angeordnet. Dieses äußere Verriegelungselement 35 dient insbesondere zur mechanischen Befestigung einer distalen Verschlusskappe, wie sie zur Figur 4 näher beschrieben wird. Daneben kann dieses äußere Verriegelungselement 35 jedoch auch zur mechanischen Befestigung bzw. Fixierung der Aufnahmeeinrichtung 30 an einer Spritze oder einem anderen Druck erzeugenden Element dienen.

Der in der Aufnahmeeinrichtung 30 fest angeordnete Schlauch 10 weist in seinem Inneren das Biegeelement 20 auf. Dieses Biegeelement 20 ist in einem komplementären Hohlraum 21 im Schlauch 10 angeordnet. Diesbezüglich wird auf Figur 2 verwiesen, in der erkennbar ist, dass das Biegeelement 20 nicht im Hohlraum des Schlauches angeordnet ist, sondern in der Schlauchwand 11.

In einer bevorzugten Ausführungsform erstreckt sich der komplementäre Hohlraum 21 über die gesamte Länge des Schlauches 10, und somit auch bis zu seinem proximalen Ende 12. Das Biegeelement 20 endet jedoch an der maximalen Erstreckungsposition 22. Der verbleibende komplementäre Hohlraum 23 ist vorzugsweise mit einer Klebemasse 24 ausgefüllt. Eine derartige Ausgestaltung hat den Vorteil der einfachen und kostengünstigen Fertigung bei Vermeidung von Verunreinigungen im komplementären Hohlraum 21.

Vorzugsweise ist der Schlauch 10 aus einem transparenten Material und das Biegeelement aus einem Titan-Draht hergestellt. Das Biegeelement 20 weist vorzugsweise einen runden Querschnitt auf.

Durch die Anordnung des Biegeelementes 20 im Schlauch 10 lässt sich der Schlauch manuell plastisch verbiegen und somit der Form eines Behandlungskanals oder der Position des Behandlungsortes im Gewebe anpassen. Ein Titan-Draht hat dabei den Vorteil, dass er bei einer gleichzeitigen MRT-Untersuchung sichtbar ist, so dass insgesamt eine sehr einfache und gut kontrollierbare Positionierung des Schlauches im Körper 10 möglich ist.

Zur optimalen Behandlung von Knorpeln lässt sich die im Schlauch 10 aufgenommene Substanz 90 in diesen Knorpel einbringen.

Zur Aufnahme der Substanz 90 in den Schlauch 10 wird eine nicht dargestellte Spritze mit ihrer Spritzendüse in den Innenkonus 31 der Aufnahmeeinrichtung 30 eingesteckt und ein Unterdruck in der Spritze erzeugt. Der Filter 40 ist gasdurchlässig und derart ausgestaltet, dass er für die Substanz 90 undurchlässig ist. Somit lässt sich die Substanz 90 in den Schlauch 10 bis zu dessen vollständiger Befüllung saugen.

Zur Abgabe der Substanz 90, zum Beispiel in biologisches und oder tierisches Gewebe, wird in der an die Aufnahmeeinrichtung 30 angeschlossenen Spritze ein Überdruck erzeugt, so dass die Substanz 90 aus dem Schlauch 10 herausgeschoben wird.

Dabei muss das Befüllen des Schlauches 10 mit der Substanz 90 und das Ausstoßen der Substanz 90 aus dem Schlauch 10 nicht unbedingt mit einem kurzen Zeitabstand erfolgen, sondern es kann der Schlauch 10 auch mit der Substanz 90 befüllt werden und über einen längeren Zeitraum gelagert und/oder über eine größere Entfernung zu einem Behandlungsort transportiert werden.

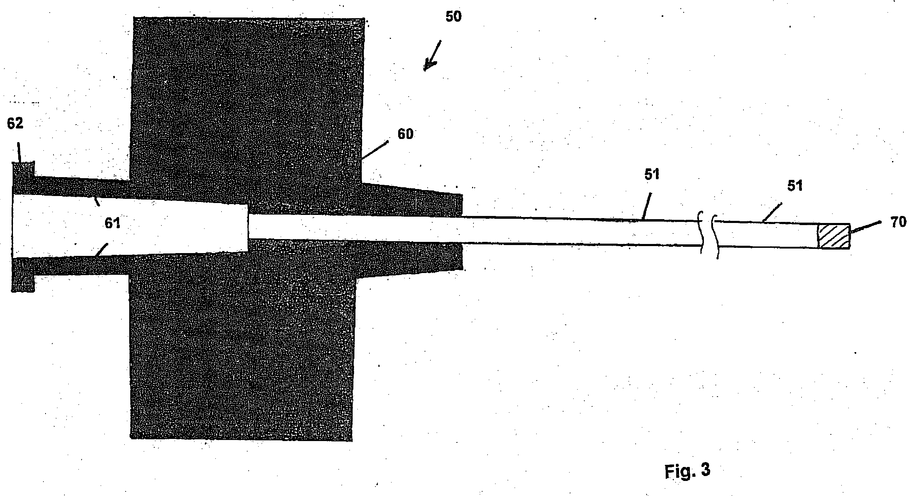

Zum Verschluss des Schlauches 10 sowie zum Schutz der darin gelagerten Substanz 90 vor Umwelteinflüssen und/oder vor Verschmutzungen ist die in Figur 3 dargestellte Schlauchhülle 50 vorgesehen. Diese Schlauchhülle 50 umfasst einen Mantelbereich 51, der im Wesentlichen hohlzylinderförmig ausgestaltet ist. Der Mantelbereich 51 ist mit einem Stopfen 70 am proximalen Ende verschlossen. Am distalen Ende weist die Schlauchhülle 50 eine Befestigungseinrichtung 60 auf. Ein Bestandteil dieser Befestigungseinrichtung 60 ist ein Innenkonus 61, der auf den Außenkonus 32 der Aufnahmeeinrichtung 30 schiebbar ist. Von dem den Innenkonus 61 ausbildenden Formelement steht radial ein äußeres Verriegelungselement 62 ab. Dieses äußere Verriegelungselement 62 dient zur Befestigung der Schlauchhülle 50 an der Aüfnahmeeinrichtung 30. Um eine formschlüssige Fixierung der Befestigungseinrichtung 60 an der Aufnahmeeinrichtung 30 zu realisieren, weist die in Figur 1 dargestellte Aufnahmeeinrichtung 30 ein Umfangselement 33, welches sich an das Formelement zur Ausgestaltung des Innenkonus 31 anschließt, auf, an dem nach innen sich radial erstreckend ein inneres Verriegelungselement 34 angeordnet ist. Dieses innere Verriegelungselement 34 kann mit dem äußeren Verriegelungselement 62 der Befestigungseinrichtung 60 derart zusammenwirken, dass die Befestigungseinrichtung 60 formschlüssig an der Aufnahmeeinrichtung 30 fixierbar ist.

In Figur 4 ist dieser Zustand der Fixierung der Befestigungseinrichtung 60 der Schlauchhülle 50 an der Aufnahmeeinrichtung 30 des Applikators 1 dargestellt. Es ist ersichtlich, dass das äußere Verriegelungselement 62 das innere Verriegelungselement 34 hintergreift, so dass eine translatorische Verschiebung der Befestigungseinrichtung 60 in Richtung des proximalen Endes 12 verhindert wird. Somit ist die Schlauchhülle 50 fest am Applikator 1 bzw. auf dem Schlauch 10 angeordnet, so dass der Schlauch 10 durch den Mantelbereich 51 der Schlauchhülle 50 sowie durch den Stopfen 70 luftdicht verschlossen ist.

Dabei sind die Länge der Schlauchhülle 50 sowie das Abstandsmaß des proximalen Endes des Schlauches 12 bis zum inneren Verriegelungselement 34 bzw. bis zum Außenkonus 32 derart aufeinander abgestimmt, dass bei Befestigung der Schlauchhülle 50 auf dem Applikator 1 der Stopfen 70 der Schlauchhülle 50 auf das proximale Ende 12 drückt. Bei Auswahl eines elastischen Materials des Stopfens 70, wie z.B. Silikon, gewährleistet die Elastizität des Stopfens eine ständige Anlage des Stopfens 70 am proximalen Ende 12, so dass ein Austreten der Substanz 90 aus dem Schlauch 10 zuverlässig verhindert wird.

Am distalen Ende 13 des Applikators 1 weist dieser, wie bereits beschrieben, das radial sich erstreckende äußere Verriegelungselement 35 auf, welches in Formschluss mit einer distalen Verschlusskappe 80 bringbar ist. Diese distale Verschlusskappe 80, welche in Figur 4 zur besseren Übersichtlichkeit vom Applikator 1 getrennt dargestellt ist, dient zum Verschluss des distalen Endes 13 des Applikators 1 und somit zur Verhinderung der Verschmutzung oder Beschädigung des Filters 40 sowie der im Schlauch 10 aufgenommenen Substanz 90.

Zur besseren Übersichtlichkeit ist der Schlauch 10 sowie die Schlauchhülle 50 gebrochen dargestellt. Für den Fachmann ersichtlich ist, dass sich das in Figur 4 unten dargestellte proximale Ende 12 der Schlauchhülle 50 sowie des Schlauches 10 eigentlich in Richtung der gemeinsamen Längsachse des Schlauches 10 sowie der Schlauchhülle 50 an diese Bauteile anschließt.

Die vorliegende Erfindung ist nicht auf die dargestellten Konusse 31, 32 und 61 sowie die daran angeordneten Elemente 34, 35 und 62 eingeschränkt, sondern es können stattdessen auch andere Verschlussarten wie zum Beispiel Gewinde- oder BajonettVerschlüsse und/oder so genannte Luer-Locks verwendet werden.

Vorteilhafterweise bilden der Außenkonus 32 der Aufnahmeeinrichtung 30 und der Innenkonus 61 der Befestigungseinrichtung 60 zusammen mit den Verriegelungselementen ein Luer-Lock aus.

### Bezugszeichenliste

- 1: Applikator
- 10: Schlauch
- 11: Schlauchwand
- 12: proximales Ende
- 13: distales Ende
- 20: Biegeelement
- 21: Komplementärer Hohlraum
- 22: Maximale Erstreckungsposition
- 23: verbleibender komplementärer Hohlraum
- 24: Klebemasse
- 30: Aufnahmeeinrichtung
- 31: Innenkonus
- 32: Außenkonus
- 33: Umfangselement
- 34: Inneres Verriegelungselement
- 35: Äußeres Verriegelungselement
- 40: Filter
- 50: Schlauchhülle
- 51: Mantelbereich
- 60: Befestigungseinrichtung
- 61: Innenkonus
- 62: Äußeres Verriegelungselement
- 70: Stopfen
- 80: distale Verschlusskappe
- 90: Substanz

## Patentansprüche

1. Applikations-Set, umfassend:
(i) einen Applikator (1)
zur zumindest temporären Aufnahme und Applikation einer Substanz (90), insbesondere zur Applikation einer Zell-haltigen und/oder gelförmigen oder
flüssigen Substanz (90) in tierische oder menschliche Organe oder Gewebe,
wobei der Applikator (1) eine distale Aufnahmeeinrichtung (30) an dem ein äußeres Verriegelungselement (35) angeordnet ist sowie einen manuell biegbaren Schlauch (10) umfasst, wobei
mit dem Schlauch (10) mechanisch wenigstens ein manuell plastisch biegbares Biegeelement (20) verbunden ist,
mit welchem nach Einwirkung eines auf den Applikator (1) wirkenden Biegemomentes der Schlauch (10) in einem verformten Zustand haltbar ist,
und
(ii) eine Schlauchhülle (50), die einen Mantelbereich (51) sowie eine mit dem Mantelbereich (51) mechanisch verbundene Befestigungseinrichtung (60) zur mechanischen Befestigung der Schlauchhülle (50) am Verriegelungselement (34, 35) des Applikators (1) aufweist, **dadurch gekennzeichnet,**
**dass** der Schlauch (10) des Applikators (1) zumindest teilweise vom Mantelbereich (51) der Schlauchhülle (50) umschlossen ist sowie der Mantelbereich (51) der Schlauchhülle (50) am proximalen Ende (12) mit einem Stopfen (70) luftdicht verschlossen ist, wobei der Durchmesser, die Länge sowie die Elastizität der Schlauchhülle (50) und des Schlauches (10) derart bemessen sind, dass zwischen der Schlauchhülle (50) und dem Schlauch (10) des Applikators (1) eine leichte Presspassung besteht, **dadurch gekennzeichnet, dass** ein fester, wasserdichter, proximaler Verschluss des Applikators (1) durch die Schlauchhülle (50) erreicht wird.

2. Applikations-Set nach Anspruch 1,
**dadurch gekennzeichnet,**
**dass** die distale Aufnahmeeinrichtung (30) des Applikators (1) zur Aufnahme einer Spritzendüse dient, so dass bei Druckerhöhung bzw. Druckverringerung in einer an die Aufnahmeeinrichtung (30) angeschlossenen Spritze der Schlauch (10) mit der Substanz (90) zumindest teilweise entleer- bzw. befüllbar ist.

3. Applikations-Set nach Anspruch 1 und 2, **dadurch gekennzeichnet, dass** die Schlauchinnenseite des Applikators (1) zumindest aus einem Material gefertigt ist, welches eine auf Grund chemischer und/oder biologischer Reaktionen hervorgerufenen Anhaftung der Substanz beziehungsweise der Zelltransplantate am Schlauch (10) verhindert.

4. Applikations-Set nach Anspruch 1,
**dadurch gekennzeichnet,**
**dass** das Biegeelement (20) in der Schlauchwand (11) angeordnet ist.

5. Applikations-Set nach den Ansprüchen 1 bis 4,
**dadurch gekennzeichnet,**
**dass** das Biegeelement (20) in einem komplementär zu seiner Form gestalteten Hohlraum (21) im Schlauch des Applikators (1) angeordnet ist, der bis zum proximalen Ende (12) des Schlauches (10) verläuft, wobei das Biegeelement (20) vor dem proximalen Ende (12) des Schlauches (10) endet und der verbleibende komplementäre Hohlraum (21) mit einer Klebemasse (24) ausgefüllt und verschlossen ist.

6. Applikations-Set nach wenigstens einem der vorhergehenden Ansprüche,
**dadurch gekennzeichnet,**
**dass** das Biegeelement (20) wenigstens ein Draht aus Titan oder einer geeigneten Titanlegierung umfasst.

7. Applikations-Set nach wenigstens einem der vorhergehenden Ansprüche,
**dadurch gekennzeichnet,**
**dass** der Applikator (1) wenigstens einen mit dem Schlauch (10) strömungstechnisch verbundenen Filter (40) umfasst, der gasdurchlässig ist.

8. Applikations-Set nach Anspruch 7,
**dadurch gekennzeichnet,**
**dass** der Filter (40) am distalen Ende (13) des Schlauches (10) angeordnet ist.

9. Applikations-Set nach wenigstens einem der vorhergehenden Ansprüche,
**dadurch gekennzeichnet,**
**dass** der Schlauch (10) zumindest teilweise mit einer zu injizierenden Substanz (90) gefüllt ist.

10. Applikations-Set nach Anspruch 1,
**dadurch gekennzeichnet,**
**dass** der Mantelbereich (51) der Schlauchhülle (50) zumindest abschnittsweise aus einem durchsichtigen Material besteht.

11. Applikations-Set nach wenigstens einem der vorhergehenden Ansprüche,
**dadurch gekennzeichnet,**
**dass** der Mantelbereich (51) der Schlauchhülle (50) durch einen Hohlzylinder ausgebildet ist, der am proximalen Ende (12) mit einem Stopfen (70) verschlossen ist.

12. Applikations-Set nach wenigstens einem der vorhergehenden Ansprüche,
**dadurch gekennzeichnet,**
**dass** die Befestigungseinrichtung (60) Bestandteil eines Luer-Lock ist.

13. Implantationsbesteck, umfassend mindestens ein Applikations-Set nach einem der vorhergehenden Ansprüche,
**dadurch gekennzeichnet,**
**dass** das Implantationsbesteck eine sterile und im Wesentlichen hermetisch abdichtende Verpackung aufweist und das Applikations-Set in der Verpackung aufgenommen ist.

## Claims

1. An application set, comprising:
(i) an applicator (1)
for at least temporarily receiving and applying a substance (90), in particular for applying a cell-containing and/or gel-like and/or liquid substance (90) into animal or human organs or tissues, the applicator (1) comprising a distal receiving device (30), on which an outer locking element (35) is disposed, and a manually bendable hose (10),
to the hose (10) being connected at least one manually plastically bendable bending element (20),
by way of which the hose (10) can be maintained in a deformed state after a bending moment acting on the applicator (1) has been applied, and
(ii) a hose sleeve (50), which comprises a jacket area (51) and a fastening device (60) mechanically connected to the jacket area (51) for mechanically fastening the hose sleeve (50) to the locking element (34, 35) of the applicator (1), **characterized in that**
the hose (10) of the applicator (1) is at least partially enclosed by the jacket area (51) of the hose sleeve (50), and the jacket area (51) of the hose sleeve (50) is closed at the proximal end (12) in an air-tight manner by a plug (70), wherein the diameter, the length and the elasticity of the hose sleeve (50) and of the hose (10) are designed such that a slight press fit exists between the hose sleeve (50) and the hose (10) of the applicator (1), whereby a fixed, waterproof, proximal closure of the applicator (1) by the hose sleeve (50) is achieved.

2. The application set according to claim 1,
**characterized in that**
the distal receiving device (30) of the applicator (1) is used to receive a syringe nozzle, so that, when the pressure is increased or the pressure is decreased in a syringe connected to the receiving device (30), the hose (10) can be at least partially emptied or filled with the substance (90).

3. The application set according to claims 1 and 2,
**characterized in that**
the hose inside of the applicator (1) is made of at least one material that prevents adhesion of the substance, or of the cell transplants, to the hose (10) caused by chemical and/or biological reactions.

4. The application set according to claim 1,
**characterized in that**
the bending element (20) is disposed in the hose wall (11).

5. The application set according to claims 1 to 4,
**characterized in that**
the bending element (20) is disposed in a cavity (21) in the hose of the applicator (10) which has a shape that complements that of the bending element and runs to the proximal end (12) of the hose (10), wherein the bending element (20) ends in front of the proximal end (12) of the hose (10), and the remaining complementary cavity (21) is filled and closed with an adhesive compound (24).

6. The application set according to at least one of the preceding claims,
**characterized in that**
the bending element (20) comprises at least one wire made of titanium or a suitable titanium alloy.

7. The application set according to at least one of the preceding claims,
**characterized in that**
the applicator (10) comprises at least one filter (40) that is fluidically connected to the hose (10) and is gas-permeable.

8. The application set according to claim 7,
**characterized in that**
the filter (40) is disposed at the distal end (13) of the hose (10).

9. The application set according to at least one of the preceding claims,
**characterized in that**
the hose (10) is at least partially filled with a substance (90) to be injected.

10. The application set according to claim 1,
**characterized in that**
the jacket area (51) of the hose sleeve (50) is made of a transparent material at least in sections.

11. The application set according to at least one of the preceding claims,
**characterized in that**
the jacket area (51) of the hose sleeve (50) is formed by a hollow cylinder, which is closed at the proximal end (12) by a plug (70).

12. The application set according to at least one of the preceding claims,
**characterized in that**
the fastening device (60) is an integral part of a Luer lock.

13. A set of implantation instruments, comprising at least one application set according to any one of the preceding claims,
**characterized in that**
the set of implantation instruments comprises a sterile and substantially hermetically sealed packaging, and the application set is accommodated in the packaging.

## Revendications

1. Kit pour application, comprenant :
(i) un applicateur (1)
pour au moins une réception temporaire et une application d'une substance (90), notamment pour l'application d'une substance (90) contenant des cellules, et/ou sous forme de gel ou de liquide dans des organes animaux ou humains, ou des tissus, où l'applicateur (1) comprend un dispositif de réception (30) distal sur lequel est disposé un élément de verrouillage (35) externe ainsi qu'un tuyau (10) pouvant être plié manuellement, où
au moins un élément en matière synthétique pouvant être plié (10) manuellement est relié mécaniquement avec le tuyau,
avec lequel, après l'action d'un moment de flexion agissant sur l'applicateur (1), le tuyau (10) peut être conservé dans un état déformé, et
(ii) une gaine de tuyau (50), qui présente une partie d'enveloppe (51) ainsi qu'un dispositif de fixation (60) relié mécaniquement avec la partie d'enveloppe (51) pour la fixation mécanique de la gaine de tuyau (50) sur l'élément de verrouillage (34, 3) de l'applicateur (1), **caractérisé en ce**
**que** le tuyau (10) de l'applicateur (1) est entouré au moins partiellement de la partie d'enveloppe (51) de la gaine de tuyau (50), de même, la partie d'enveloppe (51) de la gaine de tuyau (50) est fermée de manière étanche à l'air avec un bouchon (70) à l'extrémité proximale (12), où le diamètre, la longueur ainsi que l'élasticité de la gaine de tuyau (50) et du tuyau (10) sont dimensionnés de sorte qu'entre la gaine de tuyau (50) et le tuyau (10) de l'applicateur (1), il reste un ajustement serré, **caractérisé en ce qu'**une fermeture solide, étanche à l'eau, proximale de l'applicateur (1) est réalisée par la gaine de tuyau (50).

2. Kit pour application selon la revendication 1,
**caractérisé en ce**
**que** le dispositif de réception (30) distal de l'applicateur (1) sert au logement d'une buse de pulvérisation, de sorte qu'en cas d'augmentation de la pression, respectivement, en cas de réduction de la pression, dans un pulvérisateur associé au dispositif de réception (30), le tuyau (10) peut être au moins partiellement vidé, respectivement, rempli avec la substance (90).

3. Kit pour application selon la revendication 1, ou 2, **caractérisé en ce que** la face interne du tuyau de l'applicateur (1) est fabriquée au moins dans un matériau, lequel empêche une adhésion de la substance, respectivement, de transplants cellulaires, engendrée en raison de réactions chimiques et/ou biologiques, sur le tuyau (10).

4. Kit pour application selon la revendication 1,
**caractérisé en ce**
**que** l'élément de pliage (20) est disposé sur la paroi du tuyau (11).

5. Kit pour application selon les revendications 1 à 4,
**caractérisé en ce**
**que** l'élément de pliage (20) est disposé dans un espace creux (21), complémentaire dans sa forme, dans le tuyau de l'applicateur (1), qui s'étend jusqu'à l'extrémité proximale (12) du tuyau (10), où l'élément de pliage (20) s'arrête avant l'extrémité proximale (12) du tuyau (1) et l'espace creux (21) complémentaire restant est rempli et obturé avec une masse adhésive (24).

6. Kit pour application selon au moins l'une des revendications précédentes,
**caractérisé en ce**
**que** l'élément de pliage (20) comprend au moins un fil en titane ou dans un alliage de titane approprié.

7. Kit pour application selon au moins l'une des revendications précédentes,
**caractérisé en ce**
**que** l'applicateur (1) comprend au moins un filtre (40), qui est perméable aux gaz, relié de manière aérodynamique avec le tuyau (10).

8. Kit pour application selon la revendication 7,
**caractérisé en ce**
**que** le filtre (40) est disposé à l'extrémité distale (13) du tuyau (10).

9. Kit pour application selon au moins l'une des revendications précédentes,
**caractérisé en ce**
**que** le tuyau (10) est rempli au moins partiellement avec une substance (90) injectable.

10. Kit pour application selon la revendication 1,
**caractérisé en ce**
**que** la partie d'enveloppe (51) de la gaine de tuyau (50) est constituée au moins partiellement d'un matériau transparent.

11. Kit pour application selon au moins l'une des revendications précédentes,
**caractérisé en ce**
**que** la partie d'enveloppe (51) de la gaine de tuyau (50) est formée par un cylindre creux qui est fermé avec un bouchon (70) à l'extrémité proximale (12).

12. Kit pour application selon au moins l'une des revendications précédentes,
**caractérisé en ce**
**que** le dispositif de fixation (60) est un constituant d'un raccord Luer-Lock.

13. Instrument d'implantation, comprenant au moins un kit pour application selon l'une des revendications précédentes,
**caractérisé en ce**
**que** l'instrument d'implantation présente un emballage stérile et dans l'ensemble étanche hermétiquement et le kit pour application est logé dans l'emballage.
